# FASCICULE DE BREVET EUROPEEN

(11) **EP 1 599 436 B1**
(45) Date de publication et mention de la délivrance du brevet: **10.06.2009**
(21) Numéro de dépôt: 04716264.9
(22) Date de dépôt: 02.03.2004
(51) Int. Cl.: C07C 51/27, C07C 53/21

(54) **PROCEDE DE PREPARATION DE COMPOSES ALPHA, BETA-DIFLUOROACRYLIQUES**
VERFAHREN ZUR HERSTELLUNG VON ALPHA, BETA-DIFLUORACRYLATVERBINDUNGEN
METHOD OF PREPARING ALPHA, BETA-DIFLUOROACRYLIC COMPOUNDS

(30) Priorité: 04.03.2003 FR 0302638
(43) Date de publication de la demande: 30.11.2005
(73) Titulaire: CENTRE NATIONAL DE LA RECHERCHE SCIENTIFIQUE (CNRS), 75016 Paris (FR); Ecole Nationale Supérieure de Chimie de Montpellier, 34296 Montpellier (FR)
(72) Inventeur: AMEDURI, Bruno, 34000 Montpellier (FR); BOUTEVIN, Bernard, F-34090 Montpellier (FR); GUIOT, Jérôme, 30380 Saint Christol Les Ales (FR)
(74) Mandataire: Domenego, Bertrand
(86) Numéro de dépôt international: PCT/FR2004/000482
(87) Numéro de publication internationale: WO 2004/081169

(56) Documents cités:
- YAKUBOVICH, A. ET AL: "2,3-difluoroacrylic acid and its derivatives" JOURNAL OF GENERAL CHEMISTRY USSR (ENGL. TRANSL.), vol. 35, no. 3, 1965, pages 468-471, XP008025118 cité dans la demande
- BOGUSLAVSKAYA, L.S.: "Reactions of chlorine monofluoride. I. Electrophilic addition of chlorine monofluoride to substituted alkenes" JOURNAL ORGANIC CHEMISTRY USSR (ENGL. TRANSL.), vol. 16, 1980, pages 215-2162, XP008025117
- GUIOT, ET AL: "Synthesis of fluorinated telomers Part 7. Telomerization of 1,1-difluoro-2-chloroethylene and 1,2-difluoro1,2 dichloroethylene with methanol" NEW JOURNAL OF CHEMISTRY, vol. 26, 2002, pages 1768-1773, XP008025115 cité dans la demande
- BOGUSLOVSKAYA, L.S. ET AL: "Conjugated halogenation of unsaturated compounds I. Chlorofluorination and methoxychlorination of allyl alcohol" JOURNAL ORGANIC CHEMISTRY USSR (ENGL. TRANSL.), vol. 5, no. 9, 1969, pages 1879-1883, XP008025113 cité dans la demande

## Description

La présente invention concerne un procédé pour la préparation d'acide α,β-difluoroacrylique et de α,β-difluoroacrylates d'alkyle.

Il est connu de télomériser un composé CFC1=CFC1 avec CH₃OH par voie radicalaire amorcée par un amorceur radicalaire, par un rayonnement γ ou par un rayonnement UV [J. Guiot, et al., (New J. Chem. 2002, 26, 1768-1773 . Synthesis of fluorinated telomers ; telomerization of 1,1-difluoro-2-chloroethylene and 1,2-difluoro-1,2-dichloroethylene with methanol)].

Il est également connu que l'alcool HO-CH₂-CFH-CH₂Cl peut être oxydé en acide à l'aide d'acide nitrique, notamment par L. S. Boguslovskaya, et al, [Zhurnal Organicheskoï Khimii (version anglaise), vol. 5, n° 9, pp. 1879-1882, 1969].

L'acide α,β-difluoroacrylique et les α,β-difluoroacrylates d'alkyle peuvent être préparés à partir de trifluoroéthylène, par un procédé en sept étapes décrit notamment par A. Y. Yakubovich, et al. [Zh. Obsch. Khim. Vol. 35, n° 3, (1965) 468-471]. Le schéma réactionnel est le suivait : 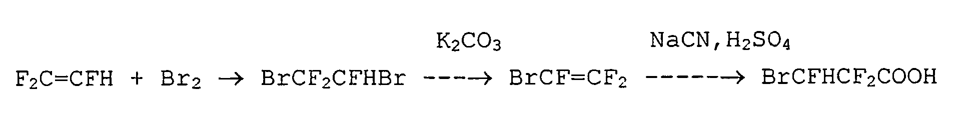 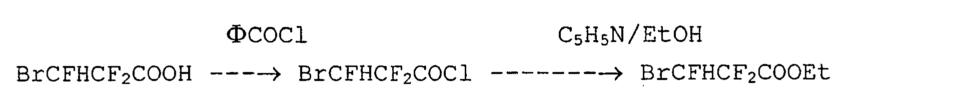 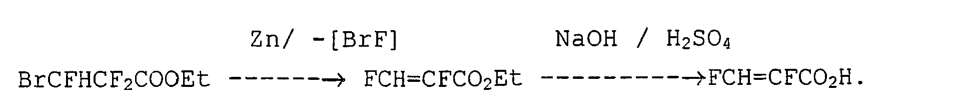 Il s'agit d'un procédé long qui met en oeuvre une grande quantité de Zn dans l'étape de déshalogénation de l'ester, et qui utilise et produit des composés à caractère explosif tels que F₂C=CFH et BrCF=CF₂.

L'acide α,β-difluoroacrylique peut en outre être préparé à partir de trifluoroéthylène, par un procédé en six étapes décrit notamment par R. Sauvêtre, et al., [Compte Rendu Acad. Sci. Paris t. 288 (19 mars 1979) Série C, pp. 335-338. Le schéma réactionnel est le suivait : 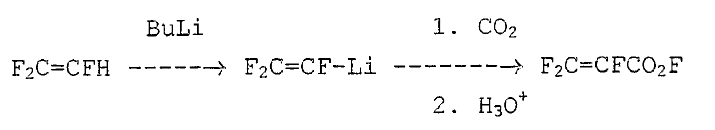 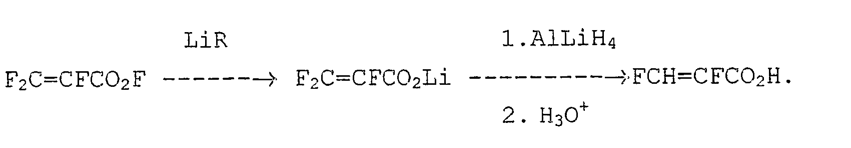 Outre le grand nombre d'étapes requises, ce procédé est peu intéressant dans la mesure où il met en oeuvre des produits dont la manipulation peut être dangereuse, tels que BuLi, LiR et ALLiH₄.

Le but de la présente invention est de fournir un procédé simple, économique et peu dangereux pour la préparation d'acide α,β-difluoroacrylique et de α,β-difluoroacrylates d'alkyle qui répondent à la formule RO-CO-CF=CFH dans laquelle R représente un atome d'hydrogène ou un groupement alkyle ayant de 1 à 5 atomes de carbone. Le procédé selon l'invention est caractérisé en ce qu'il comprend les étapes consistant à :
- préparer le 2,3-difluoro-2,3-dichloropropanol HO-CH₂-CFC1-CFC1-H (I) par télomérisation de CFC1=CFC1 avec CH₃OH, par voie radicalaire ;
- oxyder le composé (I) avec un agent oxydant pour obtenir l'acide 2,3-difluoro-2,3-dichloropropanoïque HO-CO-CFC1-CFCl-H (II)
- traiter l'acide (II) à l'aide de Zn pour déchlorer le groupe -CFCl-CFCl- en groupe -CF=CF- ;
- lorsque R est un alkyle, estérifier la fonction acide avant ou après l'étape de déchloration.

Un autre objet de l'invention est l'acide 2,3-difluoro-2,3-dichloropropanoïque (II) obtenu à la fin de la deuxième. étape du procédé.

La télomérisation peut être amorcée par un amorceur radicalaire, par un rayonnement γ ou un rayonnement UV. Lorsque la télomérisation est effectuée à l'aide d'un amorceur radicalaire; celui-ci peut être choisi par exemple parmi le 2,5-bis(tert-butylperoxy)-2,5-diméthylhexane (DHBP) le peroxyde de tert-butyle (DTBP), le peroxyde de benzoyle, le peroxypivalate de tertiobutyle, l'hydroperoxyde de tertiobutyle. La température est comprise de préférence entre 60 et 180 °C, et le rapport molaire MeOH/ CFCl=CFCl est compris de préférence entre 15 et 50.

Pour l'oxydation du composé (I), l'agent oxydant est choisi de préférence parmi l'acide nitrique, l'oxyde de chrome, l'oxyde de ruthénium et le permanganate de potassium. La durée de l'oxydation dépend de la température et de la concentration en agent oxydant. Une concentration élevée en agent oxydant permet de réduire la température et la durée de la réaction. L'acide nitrique est particulièrement préféré. L'étape b) est effectuée avantageusement en l'absence de solvant.

L'étape de déchloration est effectuée en solution dans un solvant organique, choisi de préférence parmi le DMF, le méthanol et l'éthylène glycol. On utilise le zinc de préférence sous forme de poudre de zinc. La température est comprise de préférence pour cette étape entre 30°C et 80°C. La proportion molaire de zinc par rapport au composé à déchlorer est comprise de préférence entre 1,8 et 3,2, plus particulièrement entre 2,2 et 2,8.

Pour l'obtention des esters de l'acide α,β-difluoroacrylique, l'estérification peut être effectuée soit sur l'acide HO-CO-CFC1-CFC1-H (II) avant l'étape de déchloration, soit sur l'acide α,β-difluoroacrylique HO-CO-CF=CFH obtenu après déchloration. Le mode de réalisation consistant à estérifier l'acide saturé (II) pour obtenir l'ester saturé RO-CO-CFCl-CFCl-H puis à déchlorer ledit ester est particulièrement préféré, car il supprime le risque d'une éventuelle polymérisation de l'acide HO-CO-CF=CFH ou de l'ester RO-CO-CF=CFH.

L'estérification de l'acide insaturé HO-CO-CF=CFH est effectuée à l'aide de l'alcool ROH. L'estérification de l'acide saturé (II) peut être effectuée soit directement à l'aide de l'alcool ROH, soit en transformant l'acide (II) en chlorure d'acide à l'aide de chlorure de thionyle; puis en faisant réagir le chlorure d'acide avec l'alcool ROH.

La présente invention est illustrée plus en détails par référence aux exemples suivants, auxquels elle n'est cependant pas limitée.

Les produits obtenus ont été soumis à des analyses par RMN ¹H et ¹⁹F, réalisées à température ambiante sur des appareils Bruker AC 200 et AC 250. Le solvant deuterié est le CDCl₃. La référence interne est le TMS pour la RMN ¹H, et le CFCl₃ pour la RMN ¹⁹F. Les constantes de transfert et les déplacements chimiques sont donnés respectivement en hertz (Hz) et ppm.

### Exemple 1

### Préparation de l'acide 2,3-difluoro-2,3-dichloropropanoïque

Au cours d'une première étape, on a préparé le composé HOCH₂CFC1-CFC1-H par télomérisation du composé CFCl=CFCl avec CH₃OH. On a utilisé un autoclave Parr Systems muni d'une vanne d'entrée et d'une vanne de sortie, d'un manomètre, d'un disque de rupture, d'un agitateur mécanique et d'un moyen de chauffage électrique. On a introduit dans l'autoclave 21,8 g (0,075 mol) de 2,5-bis(tert-butylperoxy)-2,5-diméthylhexane (DHBP) et 975 g (30,4 mol) de méthanol. Ensuite on a fermé l'autoclave et on l'a purgé sous une pression de 20 bars d'azote, avant d'ajouter 96,56 g (0,726 mol) de CFCl=CFCl. Le mélange a été chauffé à 134°C sous agitation, pendant 7 heures. Ensuite, l'autoclave a été refroidi jusqu'à température ambiante, puis placé dans un bain de glace. Le produit obtenu sous forme d'un liquide incolore contient deux diastéréoisomères, α et β, dont le point d'ébullition est de 63-69°C sous 20 mm/Hg.

L'analyse RMN, effectuée sur le composé obtenu 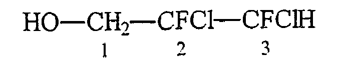 a donné les résultats suivantes :

### Diastéréoisomère α

- ¹⁹F RMN: (CDCl_{3,} ppm) δ : -130,5 (ddt, ³J_{F(F-3)} =19,5 Hz, ³J_{F(H-1)} = 15,5 Hz, ³J_{F(H-3)} = 7,0 Hz, **F**-2) ; -153,2 (ddm, ²J_{F(H}-₃₎ = 49,2 Hz, ³ J_{F(F-2)} = 19,5 Hz, **F**-3).
- ¹H RMN: (CDCl₃, ppm ) δ: 3,50 (larges, se déplaçant avec la dilution, -O**H**, 1H) ; 4,12 (m, **H**-1, 2H) 6,41 (dd, ²J_{H(F-3)} = 49,2 Hz, ³J_{H(F-2)} = 7,2 Hz, **H**-3, 1H).
- ¹³C RMN: (CDCl₃, ppm) δ : 65,12 (d, ²J_{C(F-2)} = 24,4 Hz, **C**-1) ou 65,20 (dd, ²J_{C(F-2)} = 24,4 Hz, ³J_{CF2} = 2,4 Hz, **C**-1); 97,86 (dd, ¹J_{C(F-3)} = 248,7 Hz, ²J_{C(F-2)} = 34,3 Hz, **C**-3) ou 98,29 (dd, ¹J_{C(F-3)} = 251,7 Hz, ²J_{C(F-2) =} 31,02 Hz, **C**-3) ; 110,00 (dd, ¹J_{C(F-2)} = 250,9 Hz, ²J_{C(F-3)} = 25,9 Hz, **C**-2) ou 110,70 (dd, ¹J_{C(F-2)} = 251,9 Hz, ²JC_{(F-3)} 22,4 Hz, **C**-2).

### Diastéréoisomère β

- ¹⁹F RMN: (CDCl₃, ppm ) δ : -130,9 (dddd, ³J_{F(H'-1)} = 19,6 Hz, ³J_{F(F-2)} = 19,6 Hz, ³J_{F(H''-1)} = 13,5 Hz, ³J_{F(H-3)} = 4,1 Hz, **F**-2); -148,7 (dd, ¹J_{F(H-3)} = 48,2 Hz, ³J_{F(F-2)} = 19, 6 Hz, **F**-3).
- ¹H RMN: (CDCl₃, ppm ) δ : 3,50 (large s, se déplaçant avec la dilution, O**H**, 1H) ; 4,12 (m, **H**-1, 2H) ; 6,48 (dd, ¹J_{H(F-3)} = 48,2 Hz, ³J_{H(F}-₂₎ = 4,0 Hz, **H**-3, 1H)
- ¹³C RMN: (CDCl₃, ppm) δ : 65,20 (dd, ²J_{C(F}-₂₎ 24,4 Hz, ³J_{C(F-3)} = 2,4 Hz, **C**-1) ou 65, 12 (d, ²J_{C}(F₋2) 24, Hz, **C**-1); 98,29 (dd, ¹J_{C(F-3)} = 251,7 Hz, ²J_{C(F-2)} = 31,02 Hz, **C-**3) ou 97,86 (dd, ¹J_{C(F-3)} = 248,7 Hz, ²J_{C(F}-₂₎ = 34,3 Hz, C-3) ; 110, 70 (dd, ¹J_{C(F-2)} = 251,9 Hz, ²J_{C(F-3)} = 22,4 Hz, **C**-2) ou 110,00 (dd, ¹J_{C(F-2)} = 250,9 Hz, ²J_{C(F-3)} = 25, 9 Hz, **C**-2).

Au cours d'une deuxième étape, on a préparé le composé HOOC-CFCl-CFCl-H. On a utilisé un dispositif comprenant un tricol de 250 ml muni d'un réfrigérant à boule, d'une ampoule à brome isobare, d'un barreau aimanté et d'un thermomètre. On a introduit 49,86 g (0,302 mole) de HO-CH₂-CFCl-CFCl-H dans le tricol et 101,09 g de HNO₃ (à 70 %) dans l'ampoule à brome. Ensuite on a introduit HNO₃ dans le tricol goutte à goutte sous agitation à 50°C. A la fin de l'addition, on a chauffé le mélange à 100°C pendant 24 h, puis on a refroidit le mélange obtenu qui présente une couleur verte; jusqu'à température ambiante, et on a ajouté 10 ml d'eau saturée en NaCl. Le produit de la réaction a ensuite été extrait avec 3 x 10 ml de chloroforme. Les phases chlorées ont été réunies et filtrées sur silice, le chloroforme a été évaporé et 27,0 g (0,151 mole) de HOOC-CFC1-CFC1-H ont été récupérés sous forme d'un liquide incolore avec un rendement massique de 50 %. Le composé récupéré est constitué de deux diastéréoisomères (isomère α ou β). Les résultats des analyses par RMN sont donnés ci-dessous.
- RMN ¹H: (isomère α ou β) (CDCl₃, PPm) δ : 6,82 (dd, ²J_{H(F-3)} = 46,9 Hz, ³J_{H(F-2)} = 13,8 Hz, CFCl-**H**) . 9,6 (singulet large, se déplaçant avec la dilution, -COO**H**).
- RMN ¹H: (isomère β ou α) (CDCl₃, ppm ) δ : 6,73 (dd, ²J_{H(F-3)} = 46,4 Hz, ³J_{H(F-2)} = 13,4 Hz, CFCl-**H**) . 9,6 (singulet large, se déplaçant avec la dilution, -COO**H**).
- RMN ¹⁹F: (isomère α ou β) (CDCl₃, ppm ) δ : -134,64 (dd, ³J_{F(F-3)} = 19,6 Hz, ³J_{FH} = 13,5 Hz, **F**-2), -141,12 (dd, ²J_{FH} = 45, 8 Hz, ³J_{F(F-2) =} 20,2 Hz, **F**-3).
- RMN ¹⁹F: (isomère β ou α) (CDCl₃, ppm ) δ : -135,46 (dd, ³J_{F(F-3)} = 19, 1 Hz, ³J_{FH} = 14,0 Hz, **F**-2), -153,07 (dd, ²J_{FH} = 47,0 Hz, ³J_{F(F-2)} = 19,0 Hz, **F**-3) .

### Exemple 2

### Préparation de l'acide α,β-difluoroacrylique

On a utilisé un dispositif comprenant un tricol de 100 ml contenant 24,8 g (0,38 mole) de zinc et muni d'un réfrigérant à boule, d'une ampoule à brome isobare contenant 26,6 g (0,148 mole) de HOOC-CFCl-CFCl-H (préparé selon le mode opératoire de l'exemple 1), d'un barreau aimanté et d'un thermomètre. A l'aide d'une aiguille, on a introduit 28 ml de DMF dans le tricol, puis on a ajouté très lentement sous forte agitation 1 ml (0,018 mole) de brome, puis le composé HOOC-CFCl-CFCl-H contenu dans l'ampoule à brome par petites fractions à 30 °C, ce qui a provoqué un fort exotherme. La formation de l'acide acrylique correspondant a été quasi immédiate après conversion quantitative de l'acide chlorofluorocarboxylique précurseur. Le milieu a ensuite été acidifié par 5 ml de HCl (33 %) et l'acide a été extrait par de l'éther, puis sublimé (m.p. 105°C.) sous un vide de 4.10 ₂ bars à 50 °C. On a obtenu 9,3 g (0,086 mole) de l'isomère Z sous forme de poudre blanche. Les résultats des analyses par RMN sont donnés ci-dessous.
- RMN ¹H: (CDCl₃, ppm ) δ : 7, 45 (dd, ²J_{H(F-3)} = 69,6 Hz, ³J_{H(F-2)} = 13,8 Hz, CF**H** . 9,8 (singulet large, se déplace avec la dilution, -COO**H**).
- RMN ¹⁹F: (CDCl₃, ppm ) δ : -139,45 (dd, ²J_{FH} = 69,9 Hz, ³J_{F(F-2)} = 6,3 Hz, **F**-3 , -152, 41 (dd, ³J_{FH} = 13,5 Hz, ³J_{F(F-3)} = 6,7 Hz, **F**-2).

Deux autres échantillons d'acide HOCO-CFCl-CFCl-H ont été préparés selon un mode opératoire analogue à celui décrit ci-dessus, en faisant varier la durée du chauffage lors de la deuxième étape, et ils ont été soumis à une analyse RMN ¹⁹F. Les résultats sont donnés dans le tableau 1 ci-dessous.

**Tableau 1**

| Durée du chauffage | Résultats |
|---|---|
| 3h | 55% HOCO-CFCl-CFCl-H |
| | 44% HO-CH₂-CFC1-CFC1-H |
| 7 h | 64% HOCO-CFCl-CFCl-H |
| | 36% HO-CH₂-CFCl-CFCl-H |

### Exemple 3

### Préparation du α,-β difluoroacrylate de méthyle

On a préparé de l'acide 2,3-difluoro-2,3-dichloropropionique par un mode opératoire analogue à celui de l'exemple 1, puis on a préparé le 2,3-difluoro-2,3-dichloropropionate de méthyle selon le mode opératoire suivant : dans un ballon à trois tubulures, équipé d'une ampoule à brome, d'un réfrigérant et d'un barreau aimanté, on a introduit 11,2 g (0,063 mol) d'acide 2,3-difluoro-2,3-dichloropropionique. On a ensuite ajouté goutte à goutte 19,56 g (0,145 mole) de chlorure de thionyle, puis on a chauffé à 60°C pendant 4 h. L'excès de chlorure de thionyle a ensuite été évaporé et le chlorure d'acide 2,3-difluoro-2,3-dichloropropionique distillé . On a obtenu un liquide jaune clair avec 84% de rendement.

On a versé 8,10 g (0,041 mol) du chlorure d'acide dans une solution de 6 ml de méthanol contenant 5,10 g (0,065 mol) de pyridine, puis on a chauffé le mélange au reflux du méthanol pendant 2,5 h. Après filtration du chlorure de pyridinium, lavage à l'eau, puis séchage sur sulfate de sodium, le 2,3-difluoro-2,3-dichloropropionate de méthyle a été récupéré par distillation sous forme d'un liquide incolore avec un rendement de 64%. B.P.= 143-145°C/24 mm (mélange de deux diastërêoisomères).

Les résultats de l'analyse RMN sont donnés ci-dessous.
- RMN ¹H: (isomère α ou β) (CDCl₃, ppm) δ: 3, 70 (singulet, CH₃) ; 6,84 (dd, ²J_{H(F-3)} = 46,7 Hz, ³J_{H(F-2)} = 13,7 Hz, CFCl-**H**) .
- RMN ¹H: (isomère β ou α) (CDCl₃, ppm) δ: 3,71 (singulet, CH₃) ; 6, 74 (dd, ²J_{H(F-3)} = 46,3 Hz, ³J_{H (F-2)} = 13, 4 Hz, CFCl-**H**).
- RMN ¹⁹F: (isomère αα ou β) (CDCl₃, ppm) δ: -135, 03 (dd, ³J_{F(F-3)} = 19,5 Hz, ³J_{FH} = 13,7 Hz, **F**-2), -141,22 (dd, ²J_{FH} = 45,4 Hz, ³J_{F(F-2)} = 20,0 Hz, **F**-3).
- RMN ¹⁹F: (isomère β ou α) (CDCl₃, ppm) δ: -135,34 (dd, ³J_{F(F-3)} = 19,3 Hz, ³J_{FH} = 14,2 Hz, **F**-2), -153,11 (dd, ²J_{FH} = 47,1 Hz, ³J_{F(F-2)} = 18,8 Hz, **F**-3).

La synthèse de l'α,β-difluoroacrylate de méthyle a été réalisée selon un mode expérimental similaire à celui de l'acide α,β-difluoroacrylique : à un mélange constitué par 4,82 g (0,074 mol) de zinc et 18 ml de DMF, on ajoute goutte à goutte 6,05 g (0,031 mol) de 2,3-difluoro-2,3-dichloropropionate de méthyle, puis 0,72 ml de brome, ce qui provoque un exotherme dès les premières gouttes ajoutées, à 30°C. La réaction est quasi immédiate, et après un traitement analogue à celui mis en oeuvre pour l'acide α,β-difluoroacrylique, l'ester correspondant est récupéré par distillation sous forme d'un liquide incolore avec un rendement de 56%. B.P.=24°C sous 30mm Hg. On obtient l'isomère Z. L'analyse RMN donne les résultats suivantes :
- RMN ¹H: (CDCl₃, ppm ) δ: 3, 75 (singulet, CH₃); 7,42 (dd, ²J_{H(F-3)} - 69,2 Hz, ³J_{H(F-2)} = 13,8 Hz, CF**H**)
- RMN ¹⁹F: (CDCl₃, ppm ) δ: -139,09 (dd, ²J_{FH} = 69, 5 Hz, ³J_{F(F-2)} = 6,4 Hz, **F**-3), -153, 2 (dd, ³J_{FH} = 13,3 Hz, ³J_{F(F-3)} = 6,5 Hz, **F**-2).

### Exemple 4

### Préparation de l'α, β-difluoroacrylate d'éthyle

On a préparé le chlorure de l'acide 2,3-difluoro-2,3-dichloropropionique par un mode opératoire analogue à celui de l'exemple 3, puis on a préparé le 2,3-difluoro-2,3-dichloropropionate de d'éthyle selon le mode opératoire suivant : 6,91 g (0,035 mol) du chlorure de l'acide 2,3-difluoro-2,3-dichloropropionique sont versés dans une solution de 7,5 ml d'éthanol contenant 4,35 g (0,055 mol) de pyridine, puis le mélange est chauffé au reflux de l'éthanol pendant 3 h. Après filtration du chlorure de pyridinium, lavage à l'eau puis séchage sur sulfate de sodium, le 2,3-difluoro-2,3-dichloropropionate d'éthyle est récupéré par distillation sous forme d'un liquide incolore avec un rendement de 64%. B.P.= 119-120°C/10mm (mélange de deux diastéréoisomères). Les résultats de l'analyse RMN sont donnés ci-dessous.
- RMN ¹H: (isomère α ou (3) (CDCl₃, ppm) δ : 1, 21 (triplet, ³J_{HH} =6, 9 Hz, CH₃) ; 3, 55 (quadruplet, ³J_{HH} = 7,1 Hz, Che₂) 6, 82 (dd, ²J_{H(F-3)} = 46,5 Hz, ³J_{H(F-2)} = 13, 6 Hz, CFCl-**H**) .
- RMN ¹H: (isomère β ou α) (CDCl₃, ppm) δ : 1,25 (triplet, ³J_{HH} =6.8 Hz, CH₃) ; 3,58 (quadruplet, ³J_{HH} 6, 9 Hz, CH₂) ; 6,73 (dd, ²J_{H(F-3)} = 46,2 Hz, ³J_{H(F-2)} = 13,2 Hz, CFCl-**H**).
- RMN ¹⁹F: (isomère α ou β) (CDCl_{3,} ppm) δ : -135,08 (dd, ³J_{F(F-3)} = 19,4 Hz, ³J_{FH} = 13,8 Hz, **F**-2), -141,25 (dd, ²J_{FH} = 45,2 Hz, ³J_{F(F-2)} = 20, 1 Hz, **F**-3).
- RMN ¹⁹F: (isomère β ou α) (CDCl₃, ppm) δ: -135,20 (dd, ³J_{F(F-3)} = 19, 5 Hz, ³J_{FH} = 14,1 Hz, **F**-2), -153,21 (dd, ²J_{FH} = 47,3 Hz, ³J_{F(F-2)} 18,7 Hz, **F**-3).

La synthèse de l'α,β-difluoroacrylate d'éthyle a été réalisée selon un protocole expérimental similaire à celui décrit dans l'exemple 2 pour la préparation de l'α,β-difluoroacrylate de méthyle : à un mélange constitué par 4,22 g (0,065 mol) de zinc et 12 ml de DMF, on a ajouté goutte à goutte 5,62 g (0,027 mol) de 2,3-difluoro-2,3-dichloropropionate d'éthyle, puis 0,68 ml de brome (qui entraîne un exotherme), à 30°C. Après la fin de la réaction, on a mis en oeuvre un traitement analogue à celui de la synthèse de l'α,β-difluoroacrylate de méthyle. L'α,β-difluoroacrylate d'éthyle a été récupéré par distillation sous forme de liquide incolore avec un rendement de 51% B.P.=29°C sous 34 mm Hg.

Comme précédemment, l'isomère Z est obtenu.
- RMN ¹H: (CDCl₃, ppm) δ : 1,26 (triplet, ³J_{HH} =6, 9 Hz, CH₃) ; 3,67 (quadruplet, ³J_{HH} = 7,0 Hz, CH₂) ; 7,42 (dd, ²J_{H(F-3)} = 68, 7 Hz, ³J_{H (F-2)} = 13,5 Hz, CF**H**).
- RMN ¹⁹F: (CDCl₃, ppm ) δ : -139,11 (dd, ²J_{FH} = 69,5 Hz, ³J_{F(F-2)} = 6,4 Hz, **F**-3), -153,40 (dd, ³J_{FH} = 13,3 Hz, ³J_{F(F-3)} = 6,5 Hz, **F**-2).

## Revendications

1. Procédé pour la préparation d'un composé répondant à la formule RO-CO-CF=CFH dans laquelle R représente un atome d' hydrogène ou un groupement alkyle ayant de 1 à 5 atomes de carbone, **caractérisé en ce qu'**il comprend les étapes consistant à :
- préparer le 2,3-difluoro-2,3-dichloropropanol HO-CH₂-CFC1-CFC1-H (I) par télomérisation de CFCl=CFCl avec CH₃OH, par voie radicalaire ;
- oxyder le composé (I) avec un agent oxydant pour obtenir l'acide 2,3-difluoro-2,3-dichloropropanoïque HO-CO-CFCl-CFC1-H (II) ;
- traiter l'acide (II) à l'aide de Zn pour déchlorer le groupe -CFCl-CFCl- en groupe -CF=CF- ;
- lorsque R est un alkyle, estérifier la fonction acide avant ou après l'étape de déchloration.

2. Procédé selon la revendication 1, **caractérisé en ce que** la télomérisation est amorcée par un rayonnement γ ou par un rayonnement UV.

3. Procédé selon la revendication 1, **caractérisé en ce que** la télomérisation est amorcée par un amorceur radicalaire.

4. Procédé selon la revendication 3, **caractérisé en ce que** l'amorceur radicalaire est choisi parmi le 2,5-bis(tert-butylperoxy)-2,5-diméthylhexane, le peroxyde de tert-butyle, le peroxyde de benzoyle, le peroxypivalate de tertiobutyle et l'hydroperoxyde de tertiobutyle.

5. Procédé selon la revendication 3, **caractérisé en ce que** la télomérisation est effectuée à une température comprise entre 60 et 180 °C.

6. Procédé selon la revendication 1, **caractérisé en ce que** le rapport molaire MeOH/ CFCl=CFCl est compris entre 15 et 50.

7. Procédé selon la revendication 1, **caractérisé en ce que** pour l'oxydation du composé (I), l'agent oxydant est choisi parmi l'acide nitrique, l'oxyde de chrome, l'oxyde de ruthénium et le permanganate de potassium.

8. Procédé selon la revendication 1, **caractérisé en ce que** l'étape de déchloration est effectuée en solution dans un solvant organique.

9. Procédé selon la revendication 8, **caractérisé en ce que** le solvant est choisi parmi le DMF, le méthanol et l'éthylène glycol.

10. Procédé selon la revendication 1, **caractérisé en ce que** l'étape de déchloration est effectuée à une température entre 30°C et 80°C.

11. Procédé selon la revendication 1, **caractérisé en ce que** la proportion molaire de zinc par rapport au composé à déchlorer est comprise entre 1,8 et 3,2.

12. Procédé selon la revendication 1, comprenant une étape d'estérification, **caractérisé en ce que** l'estérification est effectuée sur l'acide α,β-difluoroacrylique HO-CO-CF=CFH obtenu après déchloration, à l'aide de l'alcool ROH.

13. Procédé selon la revendication 1, comprenant une étape d'estérification, **caractérisé en ce que** l'estérification est effectuée sur l'acide HO-CO-CFCl-CFCl-H (II) avant l'étape de déchloration.

14. Procédé selon la revendication 13, **caractérisé en ce que** l'estérification de l'acide saturé (II) est effectuée en faisant réagir l'acide (II) avec l'alcool ROH.

15. Procédé selon la revendication 13, **caractérisé en ce que** l'estérification de l'acide saturé (II) est effectuée en faisant réagir le chlorure de thionyle sur l'acide (II), puis en faisant réagir le chlorure d'acide obtenu avec l'alcool ROH.

16. Composé répondant à la formule HO-CO-CFCl-CFCl-H.

## Claims

1. Method for preparing a compound corresponding to the formula RO-CO-CF=CFH in which R represents an atom of hydrogen or an alkyl group having from 1 to 5 atoms of carbon, **characterised in that** it comprises the steps involving:
- preparing the 2,3-difluoro-2,3-dichloropropanol HO-CH₂-CFC1-CFC1-H (I) by means of the telomerisation of CFC1=CFCl with CH₃OH, via a radical route;
- oxidising the compound (I) with an oxidising agent in order to obtain 2,3-difluoro-2,3-dichloropropionic acid HO-CO-CFCl-CFCl-H (II);
- processing the acid (II) using Zn in order to dechlorinate the group -CFCl-CFCl- into the group -CF=CF-;
- when R is an alkyl, esterifying the acid function before or after the dechlorination step.

2. Method according to claim 1, **characterised in that** the telomerisation is initiated by a γ ray or UV radiation.

3. Method according to claim 1, **characterised in that** the telomerisation is initiated by a radical initiator.

4. Method according to claim 3, **characterised in that** the radical initiator is selected from 2,5-bis(tert-butylperoxy)-2,5-dimethylhexane, tert-butyl peroxide, benzoyl peroxide, tertiobutyl peroxypivalate and tertiobutyl hydroperoxide.

5. Method according to claim 3, **characterised in that** the telomerisation is carried out at a temperature of between 60 and 180°C.

6. Method according to claim 1, **characterised in that** the molar ratio MeOH/CFCl=CFCl is between 15 and 50.

7. Method according to claim 1, **characterised in that**, for the oxidation of the compound (I), the oxidising agent is selected from nitric acid, chromium oxide, ruthenium oxide and potassium permanganate.

8. Method according to claim 1, **characterised in that** the dechlorination step is carried out in solution in an organic solvent.

9. Method according to claim 8, **characterised in that** the solvent is selected from DMF, methanol and ethylene glycol.

10. Method according to claim 1, **characterised in that** the dechlorination step is carried out at a temperature of between 30°C and 80°C.

11. Method according to claim 1, **characterised in that** the molar proportion of zinc relative to the compound to be dechlorinated is between 1.8 and 3.2.

12. Method according to claim 1, comprising an esterification step, **characterised in that** the esterification is carried out on the α,ß-difluoroacrylic acid HO-CO-CF=CFH obtained after dechlorination using alcohol ROH.

13. Method according to claim 1, comprising an esterification step, **characterised in that** the esterification is carried out on the acid HO-CO-CFCl-CFCl-H (II) before the dechlorination step.

14. Method according to claim 13, **characterised in that** the esterification of the saturated acid (II) is carried out by reacting the acid (II) with the alcohol ROH.

15. Method according to claim 13, **characterised in that** the esterification of the saturated acid (II) is carried out by reacting thionyl chloride with the acid (II), then by reacting the chloride of the acid obtained with the alcohol ROH.

16. Compound corresponding to the formula HO-CO-CFCl-CFCl-H.

## Patentansprüche

1. Verfahren zur Herstellung einer Verbindung gemäß der Formel RO-CO-CF=CFH, in der R ein Wasserstoffatom oder eine Alkylgruppe mit 1 bis 5 Kohlenstoffatomen repräsentiert, **dadurch gekennzeichnet, dass** es die folgenden Schritte beinhaltet:
- Herstellen des 2,3-Difluor-2,3-Dichlorpropanols HO-CH₂-CFCl-CFCl-H (I) durch Telomerisation von CFCI=CFCI mit CH₃OH auf radikalischem Weg;
- Oxidieren der Verbindung (I) mit einem Oxidationsmittel zum Erhalten der 2,3-Difluor-2,3-dichlorpropansäure HO-CO-CFCl-CFCl-H (II);
- Behandeln der Säure (II) mit Zn zum Dechlorieren der Gruppe -CFCI-CFCI- in die Gruppe -CF=CF-;
- wenn R ein Alkyl ist, Verestern der Säurefunktion vor oder nach dem Dechlorierungsschritt.

2. Verfahren nach Anspruch 1,
**dadurch gekennzeichnet, dass**
die Telomerisation durch γ-Bestrahlung oder durch UV-Bestrahlung eingeleitet wird.

3. Verfahren nach Anspruch 1,
**dadurch gekennzeichnet, dass**
die Telomerisation durch einen radikalischen Initiator eingeleitet wird.

4. Verfahren nach Anspruch 3,
**dadurch gekennzeichnet, dass**
der radikalische Initiator ausgewählt wird aus 2,5-bis(tert-Butylperoxy)-2,5-dimethylhexan, tert-Butylperoxid, Benzoylperoxid, tert-Butyl-Peroxypivalat und tert-Butyl-Hydroperoxid.

5. Verfahren nach Anspruch 3,
**dadurch gekennzeichnet, dass**
die Telomerisation bei einer Temperatur zwischen 60 und 180° durchgeführt wird.

6. Verfahren nach Anspruch 1,
**dadurch gekennzeichnet, dass**
das Molverhältnis MeOH/CFCl=CFCl zwischen 15 und 50 liegt.

7. Verfahren nach Anspruch 1,
**dadurch gekennzeichnet, dass**
zum Oxidieren der Verbindung (I) das Oxidationsmittel ausgewählt wird aus Salpetersäure, Chromoxid, Rutheniumoxid und Kaliumpermanganat.

8. Verfahren nach Anspruch 1,
**dadurch gekennzeichnet, dass**
der Dechlorierungsschritt in Lösung in einem organischen Lösungsmittel ausgeführt wird.

9. Verfahren nach Anspruch 8,
**dadurch gekennzeichnet, dass**
das Lösungsmittel ausgewählt wird aus DMF, Methanol und Ethylenglykol.

10. Verfahren nach Anspruch 1,
**dadurch gekennzeichnet, dass**
der Dechlorierungsschritt bei einer Temperatur zwischen 30°C und 80°C ausgeführt wird.

11. Verfahren nach Anspruch 1,
**dadurch gekennzeichnet, dass**
der Molanteil von Zink in Bezug auf die zu dechlorierende Verbindung zwischen 1,8 und 3,2 liegt.

12. Verfahren nach Anspruch 1, das einen Veresterungsschritt beinhaltet,
**dadurch gekennzeichnet, dass**
die Veresterung an der nach der Dechlorierung erhaltenen α,β-Difluoracrylsäure HO-CO-CF=CFH mit Hilfe des Alkohols ROH durchgeführt wird.

13. Verfahren nach Anspruch 1, das einen Veresterungsschritt beinhaltet,
**dadurch gekennzeichnet, dass**
die Veresterung an der Säure HO-CO-CFCl-CFCl-H (II) vor dem Dechlorierungsschritt durchgeführt wird.

14. Verfahren nach Anspruch 13,
**dadurch gekennzeichnet, dass**
die Veresterung der gesättigten Säure (II) erfolgt, indem Säure (II) mit dem Alkohol ROH reagieren gelassen wird.

15. Verfahren nach Anspruch 13,
**dadurch gekennzeichnet, dass**
die Veresterung der gesättigten Säure (II) erfolgt, indem Thionylchlorid auf der Säure (II) reagieren gelassen wird, dann das erhaltene Säurechlorid mit dem Alkohol ROH reagieren gelassen wird.

16. Verbindung entsprechend der Formel HO-CO-CFCl-CFCl-H.
